# EUROPEAN PATENT SPECIFICATION

(11) **EP 0 718 627 B1**
(45) Date of publication and mention of the grant of the patent: **07.07.1999**
(21) Application number: 96200278.8
(22) Date of filing: 14.03.1991
(51) Int. Cl.: G01N 33/571, G01N 33/546, G01N 33/554

(54) **A process for preparing diagnostic reagent for syphilis**
Verfahren zur Herstellung einer diagnostischen Reagenz für Syphilis
Procédé de préparation d'un réactif de diagnostic de syphilis

(30) Priority: 16.03.1990 JP 6798690
(43) Date of publication of application: 26.06.1996
(62) Divisional of application: 91400696.0
(73) Proprietor: SEKISUI CHEMICAL CO., LTD., Osaka (JP)
(72) Inventor: Ishikawa, Fumio, Takatsuki-shi, Osaka (JP); Nagahara, Kouhei, Kamaishi-si, Iwate-ken (JP); Matsumoto, Mie, Moriyam-shi, Shiga-ken (JP)
(74) Representative: Vialle-Presles, Marie José

(56) References cited:
- EP-A- 0 038 150
- EP-A- 0 079 145
- EP-A- 0 138 167
- WO-A-87/03692
- FR-A- 2 233 024
- GENITOURIN MED, vol. 62, 1986 LONDON, pages 367-372, XP 000195914 VAN EIJK ET AL. 'Enzyme linked immunosorbent assays with Treponema pallidum ....'
- Laboratory Techniques in Biochem and Mol Biol, Elsevier, ed. P. Tijssen, 1985, Chapter 13, p.297.

## Description

The present invention relates to a process for preparing a diagnostic reagent for syphilis.

Diagnostic methods have been performed which utilize the antigen-antibody reaction of TP antigens and anti-treponemal antibodies (hereinafter abbreviated to TP antibody) in sera from syphilitic patients. Among such methods, TPHA (Treponema pallidum hemaggultination assay test) has been widely used in recent years because of the advantages in its sensitivity, specificity and convenience in operation. Therefore, the TPHA has been a typical diagnostic method for syphilis.

The antigen solution originated from TP and used in the above-mentioned method is prepared as follows: First, TP is inoculated and cultivated in rabbit testes. The treponemes are extracted and suspended in a suitable buffer and then disrupted by homogenizer, sonicator and so forth. Thus disrupted treponemes with or without solubilization was used as the antigen solution for sensitization.

However, the prior art has the following drawbacks. Specifically, primary syphilis can not sufficiently be detected by the diagnostic agent for syphilis made from the conventional TP antigen solution. In other words, the conventional TPHA test or the like does not show a positive result in most cases until 2 to 3 months after syphilitic infection. Accordingly, there is a great problem that, in order to accomplish reliable diagnosis for primary syphilis, a diagnostic reagent using a lipoidal antigen (cardiolipin) should be used together with the TPHA method. Although the reagent using lipoidal antigens Is sensitive to primary syphilis, nonspecific reactions are often observed.

An immunological diagnostic reagent is generally prepared by immobilizing an antigen or antibody on a hydrophobic carrier (e.g., plastic particles such as latex particles, cellulose powder, polystyrene, polypropylene or nylon particles; membrane of nitro-cellulose or nylon; erythrocytes treated with tannic acid; or agarose gel). Known immobilization methods include a method by physical adsorption wherein an antigen or antibody is in contact with a hydrophobic carrier in an aqueous medium and a method wherein an antigen or antibody is covalently bonded to a carrier having an amino group or a carboxylic group on its surface. The former method utilizing physical adsorption is widely used in view of manufacturing efficiency, convenience and being easy to reproduce the product of the same quality.

In the case of immobilizing an antigen or antibody by physical adsorption, an approximately neutral buffer comprising a salt and a buffering agent is usually used as the aqueous medium. A surfactant is not employed as the aqueous medium, since the surfactant is considered to interfere with the immobilization of antigen or antibody on carrier. Specifically, the surfactant is considered to decrease the hydrophobic interaction in immobilization by physical adsorption.

The surfactant is also considered to interfere with the immobilization of TP antigens on carriers. In other words, the efficiency of immobilization is substantially decreased in the presence of the surfactant, with the result that it is difficult to prepare an excellent diagnostic reagent.

The object of the present invention is to provide a process for preparing a diagnostic reagent for syphilis.

The diagnostic reagent for syphilis in the present invention comprises a treponemal antigen and a carrier.

The reagent is prepared by treating a carrier with a treponemal antigen in an aqueous medium having a pH from about 4.5 to about 7.7 and containing a surfactant in an amount of from about 0.01 wt. % to about 2.5 wt. % and removing the remaining surfactant if any, the surfactant being octylglucopyranoside.

### (1) Diagnostic agent and a method for preparing the same

The treponemal antigens are immobilized on a carrier to make a reagent for diagnosing syphilis.

Inert carriers in which the surface is at least partially hydrophobic can be used. Examples of the carriers are synthetic polymer having a particle size of about 0.05 to 50 µm prepared by performing polymerization or copolymerization with a monomer such as styrene, acrylic acid, methyl methacrylate, acrylonitrile or butadiene and particularly microparticles having an uniform particle size of 0.1 to 2µm, i.e., latex particles called in the field of an immunological diagnostic reagent, prepared by emulsion-polymerizing the aforesaid polymer or its derivatives in an aqueous medium; synthetic polymer materials such as polystyrene, polyethylene, polypropylene, nylon or cellulose acetate and their molded product; membrane of nylon, nitrocellulose or the like; materials of living organism such as sheep or hen erythrocytes treated with tannic acid; and inorganic materials such as silica powder or glass particles, or the like. The latex particles and erythrocytes treated with tannic acid are more preferable.

"The surface is at least partially hydrophobic" means the property for immobilizing the antigen via any physical adsorption. The surface may entirely be hydrophobic. Some materials may be used by activating the surface thereof.

The immobilization of the antigen on a carrier is conducted in contacting them in an aqueous medium containing about 0.01 to 2.5% by weight of octylglucopyranoside (1-O-n-octyl-β-D-glucopyranoside) as a surfactant and having a pH of from about 4.5 to 7.7, thereby providing a reagent with excellent sensitivity and improved specificity in which the antigens are much stably immobilized.

Besides, in case where the surfactant is used at a high concentration, it is preferable to remove the possibly remaining surfactant after the immobilization, in order to avoid the interference of the surfactant with the antigen-antibody reaction.

The surfactant used for the above immobilization, which is octylglucopyranoside, can be used for extraction and stabilization of the surface antigen and membrane protein of TP, is capable of extracting and solubilizing the object constituents, has high specificity of extractability, and is stable at a pH of from about 4.5 to 7.7 without separation.

The effective concentration of the surfactant in the aqueous medium for immobilizing the TP antigen on the carrier is about 0.01 to 2.5 wt.%, preferably about 0.02 to 2.10 wt.%. If the concentration of the surfactant is higher, the TP antigen cannot be immobilized on the carrier. The activity of the TP antigen is likely to be lost with the surfactant of lower concentration.

Preferable examples of the aqueous media used for immobilizing the TP antigen on the carrier are buffers used for general biochemical experiments, e.g., phosphate buffer, Tris buffer or the like. The ionic strength is adjusted by the addition of salt to the aqueous medium. The effective pH is about 4.5 to 7.7, preferably about 4.9 to 7.1, more preferably about 5.4 to 6.5. If the pH is less than 5.0, antigenicity is likely to be lost, but the adsorption is performed instantaneously. Thus, the pH may be more than 4.5. The quantity of the TP antigen immobilized on the carrier becomes less with the aqueous medium of pH more than 7.7. In order to increase the storage stability of the obtained diagnostic reagent, a preservative can be added thereto if necessary.

Further, choline chloride, EDTA, saccharides (polysaccharide, dextran or the like), polyethylene glycol and the like can be added in order to improve the sensitivity in measurement.

Explained subsequently is a specific method for treating the carrier with the antigen solution.

First, the antigen solution for immobilization is prepared by any known method. For example, the treponemes are extracted from rabbit testicular materials containing treponemes. Then, the treponemes are washed, to which a surfactant is added. The resultant mixture is incubated to disrupt the treponemes and extract the TP antigen. The extract is centrifuged to collect the supernatant which is then diluted with a buffer containing (octylglucopyranoside) to obtain treponemal antigen solution. The obtained solution is adjusted to have a predetermined surfactant concentration and pH, thus making the sensitizing solution, i.e., the antigen solution for immobilization.

An antigen solution from T. Pallidum can be prepared by a process which comprises adsorbing an extract originated from T. pallidum on a hydroxyapatite gel, followed by elution, while an aqueous medium is used, as described in EP 0447 322.

Subsequently, the sentizing solution is in contact with the carrier described above in an aqueous medium containing about 0.01 to 2.5 wt.% of the surfactant and having a pH of from about 4.5 to 7.7. The 5 resultant mixture was incubated for a predetermined period to immobilize the TP antigen on the carrier.

Other methods for immobilizing the TP antigen on the carrier include various ones, e.g., a method in which an antigen solution containing a suitable concentration of the surfactant is added to a carrier suspension containing or not containing the surfactant to adjust the concentration of the surfactant and pH to a predetermined value, and then results in immobilization of the antigens, or a method in which a carrier is contacted with an antigen solution and then a diluent comprising the surfactant or buffer is added to adjust the concentration of the surfactant and pH to a predetermined value thereby immobilizing the TP antigen.

After the immobilization, the carrier is separated from the aqueous medium and then washed with a buffer containing bovine serum albumin, saline solution or the like to remove the surfactant.

The antigen sensitizing solution described above is also prepared from a so-called partially purified antigen fraction. An excellent diagnostic reagent can be obtained as mentioned below by using the treponemal antigen purified according to EP 0 447 322 with the carrier of latex particles.

The octylglucooyranoside used for immobilizing the antigen on the carrier in the present invention is considered to stabilize the antigens in the antigen solution and prevent unnecessary components in the material from being immobilized on the carrier. In general, the surfactant interferes with the immobilization of antigens on carriers. The antigens used in the process according to the present invention are slightly soluble in water and hence low isoelectric points, whereby the solubility of the antigen is decreased by lowering pH so that the immobilization on carrier occurs even in the presence of the surfactant. As a result, the diagnostic reagent of the present invention has an increased amount of the antigen carried on the carrier and more excellent sensitivity compared with that prepared by the conventional method. Further, amount of other components immobilized the carrier decreases, thereby improving specificity.

### (2) Application of the diagnostic reagent

The diagnostic agent of the present invention is used for detecting anti-treponemal antibodies in the subject serum. The detection methods include radio-immunoassay (RIA), fluorescent immunoassay (FIA), enzyme immunoassay (EIA or ELISA), latex agglutination assay, TPHA method (Treponema pallidum hemaggultination assay test) or the like. The diagnostic reagent of the present invention can be formed to suit the above-mentioned detection methods.

The immunoassay utilizing the antigen-antibody reaction is preferably conducted in the presence of a water-soluble polymer and/or water-soluble copolymer containing at least one kind of glycoside derivatives represented by the following formula (I) as a monomer: 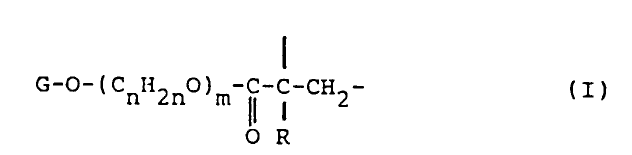 wherein G-O- represents a saccharic residue not having a protective group. R is a hydrogen atom, a methyl group or an ethyl group, m is an integer of 1 to 3 and n is an integer of 1 to 4.

The saccharic residue in the above glycoside derivative is a group in which a hydrogen atom is removed from a hydroxyl group bonded to the glycosidic carbon atom of the reduced end of a saccharide. Specifically, the saccharic residue means a residue of a monosaccharide comprising 1 to 3 sugar units or an oligosaccharide.

Examples of the monosaccharides are hexoses such as glucose, mannose, galactose, glucosamine, mannosamine or galactosamine, or pentoses such as arabinose, xylose or ribose.

Examples of the oligosaccharides are disaccharides such as maltose, lactose, trehalose, cellobiose, isomaltose, gentiobiose, melibiose, laminaribiose, chitobiose, mannobiose or sophorose; or maltotriose, isomaltotriose, taltotetraose, maltopentaose, mannotriose or manninotriose.

The polymers or copolymers containing glycoside derivatives disclosed in PCT Application No. 90/04598 can be used for the above method.

There is no limitation in the molecular weight of the polymer or copolymer containing glycoside derivativc if said polymer or copolymer is soluble in the reaction medium. A low molecular weight requires much amount of polymers or copolymers, thus taking much time to dissolve them in the reaction medium. Therefore, the molecular weight is preferably more than 3,000.

The concentration of the polymer or copolymer containing glycoside derivatives in the reaction system of the antigen-antibody reaction is suitably determined depending upon the molecular weight of the polymer or copolymer, coexisting additives such as salts, proteins, or saccharides. Generally, the polymer or copolymer is adjusted to be contained in the reaction system in an amount such that the final concentration at the time of reaction is 0.01 to 10.0 %(W/V), preferably about 0.1 to 5.0% (W/V), more preferably about 0.5 to 2.0% (W/V). When the concentration of the above polymer or copolymer is less than 0.01% (W/V), the above polymer or copolymer is less effect for accelerating the antigen-antibody reaction. On the other hand, the polymer or copolymer having more than 10.0% (W/V) of the concentration increases in nonspecific reaction with the materials other than the object constituents.

The present invention will be explained in detail hereinbelow with reference to Examples, by which no limitation shall not be given.

### Example 1 Method for purifying antigen

TP antigens were purified according to the following method :
1. Reagents and others
(1) Buffer solution
   (1-1) Phosphate buffered saline (pH:6.5)
      (hereinafter abbreviated to PBS):
      A buffer was prepared from potassium dihydrogen phosphate, sodium dihydrogen phosphate (12 hydrate) and sodium chloride to make phosphate concentration of 0.036M, NaCl concentration of 0.156M and pH 6.5. To this buffer was added NaN₃ at a concentration of 0.1% (W/V).
   (1-2) 1% BSA/PBS:
      Bovine serum albumin (hereinafter abbreviated to BSA, manufactured by Miles Laboratories Co.) was dissolved in PBS to make 1% (W/V) BSA solution.
   (1-3) 10mM potassium phosphate buffer
      (pH:6.0 and pH:7.0) (hereinafter abbreviated to KPB):
      10mM potassium dihydrogen phosphate solution was mixed with 10mM dipotassium hydrogen phosphate to obtain potassium phosphate buffers having pH 6.0 and 7.0.
   (1-4) 350mM KPB (pH:6.0):
      350mM potassium dihydrogen phosphate was mixed with 350mM dipotassium hydrogen phosphate to obtain 350mM potassium phosphate buffer having pH 6.0.
(2) Surfactant:
   Octylglucopyranoside (1-O-n-octyl-β-D-glucopyranoside, hereinafter abbreviated as OG) used for a study of slightly soluble proteins (manufactured by Nacalai Tesque, Inc.) was employed as a surfactant.
(3) Chromatographic gel for purifying protein
   (3-1) Cation exchanger
      Sepharose Fast Flow (Pharmacia LKB Biotechnology), which is cation exchanger wherein a sulfonic acid group was introduced to the surface of agarose gel, was used as cation exchanger.
   (3-2) Hydroxyapatite gel
      Bio-Gel® HTP (Bio-Rad Laboratories) and HCA-200L (Mitsui Toatsu Chemicals, Inc.) were used as hydroxyapatite gel.
(4) TP
   Treponemes were used was cultivated and isolated by the following method.
   A suspension of a pathogenic standard Nichols strain of Treponema pallidum (6.0x10⁷/ml) was inoculated in rabbit testes in an amount of 1 ml per testis. After the cultivation for 10 days, testes were taken out from 10 rabbits, sliced and then shaked for 30 minutes at 37°C in 2.2% sodium citrate solution (500ml). Thereafter, proliferated treponemes were extracted. The extract was centrifuged for 5 minutes at 200 x g to remove the precipitate of rabbit tissue. The supernatant was centrifuged for 30 minutes at 3000 x g to precipitate treponemes. Thus obtained treponemes were well washed with PBS and suspended in PBS to adjust the number of the treponemes to 1 x 10⁹ after the counting with a dark-field microscope. Thus, a suspension of TP mycelia was obtained. This suspension was confirmed with a dark-field microscope that no sperms and tissues of rabbit were included.
(5) Reagent for measuring concentration of protein:
   BCA® Protein Assay Reagent (Pierce Co.) was used as a reagent for measuring concentration of protein.
(6) Microtiter plate:
   A microplate having 96 wells (Nunc Co., U-bottom) was used.
(7) TP antigen sensitized erythrocyte:
   Used sensitized erythrocyte was the one used for Seroclit TP (The Chemo- Sero-Therapeutic Research Institute), which is a TPHA kit on market.
(8) Syphilis-positive serum of rabbit:
   Used serum was the one taken out from the rabbit which was subjected to the cultivation of TP in its testes for 45 days. Antibody titer was measured by the commercially available TPHA kit, obtaining a value of 102,400. This serum was diluted with 1% BSA/PBS for use.

2. Experimental Method
(2-1) Solubilization and extraction of antigen from TP
   The suspension of treponemes (10 ml) was washed three times with PBS (50 ml), followed by suspending in PBS (20ml). The resultant suspension was sonicated for disruption. The suspension was subjected to centrifugation for 30 minutes at 12,600 x g to take out the precipitate.
   Thus obtained precipitate was washed twice with KPB (10 mM, pH 7.0) using centrifugation for 30 minutes at 12,600 x g. Thereafter, KPB (10 mM, pH 7.0) containing OG in 1% (W/V) was added in an amount of 25 ml to the precipitate. The suspension was then slightly sonicated to be solubilized. After being left at 4°C for 16 hours or more, the mixture was centrifuged for 1 hour at 50,000 x g. The resulting supernatant was filtered through a filter of 0.22 µm (Millex-GS produced by Millipore Corporation). The extract obtained from the treponemes in this way was referred to as the extracted antigen hereinbelow.
(2-2) Pretreatment
   (1) Dialysis of antigen solution
      The extracted antigen which was dissolved in a buffer having pH 7.0 was dialyzed against a buffer having pH 6.0 and containing 1% OG. The dialysis was carried out with the volume ratio of the dialyzing solution to the extracted antigen solution being 4 to 1. The dialyzing solution was exchanged three times. After the final dialysis, the pH value of the dialyzing solution (external solution for dialysis) was confirmed to be in the range of from 6.0 ± 0.1.
   (2) The antigen obtained in (1) was passed through a column (Pharmacia LKB Biotechnology, SR 25/45) of S Sepharose Gel (30 ml) to collect 50ml of the passed-through fraction as the antigen fraction (hereinbelow referred to as the partially purified antigen).
(2-3) Purification of antigen by hydroxyapatite gel
   (1) Washing of hydroxyapatite gel
      A column (Pharmacia LKB Biotechnology, HR 10/10) was filled with the hydroxyapatite gel (8 ml), followed by equilibrated with 10mM KPB containing 1% OG (pH 6.0). The optical density (hereinafter abbreviated to O.D.) of the washing solution at 280 nm was measured. Washing was continued until the absorbance of the eluate decreases to 0.010.
   (2) Addition of the partially purified antigen
      The partially purified antigen was added to a hydroxyapatite column. Thereafter, 10mM KPB containing 1% OG (pH 6.0) was passed through the column. The column was washed until the absorbance of the eluate solution becomes 0.010 or less at 280 nm. The fraction thus obtained was defined as the passed-through fraction.
   (3) Elution of antigen
      A linear gradient elution was conducted by gradually increasing the ratio of 350mM KPB containing 1% OG to 10mM KPB containing 1% OG (pH 6.0) from 0 to 40%, and finally increasing it to 100%. The fractions of the ratios of 0 - 8%, 8 - 16%, 16 - 24%, 24 - 32% and 32 - 40% were collected. The fraction at the ratio of 100% is defined as the 40% or more fraction.
   (4) Assay of each fraction
      The antigen activity and protein concentration of each of the extracted antigen, the passed-through fraction, 0 - 8% fraction, 8 - 16% fraction, 16 - 24% fraction, 24 - 32% fraction, 32 - 40% fraction and 40% or more fraction were measured by the following methods. Specific activitiy was calculated from the antigen activity and protein concentration.
(2-4) Antigen assay method
   (1) Protein concentration
      The protein concentration was measured by BCA® protein Assay Reagent (Pierce Co.) in which a measuring method [Smith, P.K., Krohn, R.I. etc., (1985) Anal. Biochme. 150, 76-85] was used as its principle. Said measuring method uses bicinchoninic acid and is one of the modified Lawry method. The used standard was a solution of BSA in 10mM KPB containing 1% OG. The unit was expressed by µg/ml.
   (2) Antigen activity
      (a) 25µl of 1% BSA/PBS was dispersed in each well of the microtiter plate.
      (b) 25µl of each antigen fraction was dispersed in the well of the plate and 25µl of it was transferred in the above next well repeatedly to be diluted serially 2¹ to 2ⁿ fold with 1% BSA/PBS on the plate.
      (c) Syphilis-positive rabbit serum, in which the antibody titer was diluted to 50 fold, was added to and mixed with the antigen fractions diluted 2¹ to 2ⁿ fold in (b).
      (d) The mixture was incubated for 30 minutes or more at room temperature. The antibody in the well having a high concentration of antigen was consumed by antigen-antibody reaction, while the antibody on the well having a low concentration of antigen remained thereon.
      (e) Subsequently, TP antigen sensitized erythrocyte in the commercially available TPHA kit (Serodia-TP) was added to each well. A final dilution ratio of the rabbit serum which caused the hemagglutination was defined as the antigen activity. This antigen activity was represented by titer (titer/ml).
      (g) Among the fractions exhibiting the antigen activity, those exhibiting a specified activity of 12 titer/µg or more were collected. Thus collected fractions were concentrated under reduced pressure by using cellophane tube (Wako Pure Chemical Industries, Ltd.) until the protein concentration became to 50µg/ml or more. The obtained fractions were defined as the HAp purified antigen.

3. Other experiments
Antigens were purified with Bio-Gel® HTP and HCA-200L by the same manner as described above. Besides the antigen activity and protein concentration of each of the fractions, those of extracted antigen and partially purified antigen were measured, whereby total antigen activity, total amount of protein and total specific activity of antigen were calculated. Table 1 shows the results.
4. Conclusion
As is apparent from Table 1, when the TP extract was eluted after adsorbing on the hydroxyapatite gel, the antigens were eluted from Bio-Gel® HTP at a salt concentration of 8% (37.2 mM) to 40% (146.0 mM) and from HCA-200L at a salt concentration of 8% (37.2 mM) to 32% (118.8 mM). Thus obtained fractions were combined, thereby obtaining the TP antigen of high purity having specific activities of 26.6 and 36.7 (titer/µg) respectively.

**Table 1**

| (1) Purification from Bio-Gel® HTP | | | |
|---|---|---|---|
| Fractions | Total Antigen Activity (titer) | Total amount of Protein (µg) | Specified Activity of Antigen (titer/µg) |
| passed-through | 0 | 850 | -- |
| 0 - 8% | 0 | 90 | -- |
| 8 - 16% | 4610 | 170 | 27.1 |
| 16 - 24% | 9220 | 240 | 38.4 |
| 24 - 32% | 4610 | 180 | 25.6 |
| 32 - 40% | 2300 | 190 | 12.1 |
| 40% or more | 0 | 430 | -- |
| Total of 8-40% | 20740 | 780 | 26.6 |
| Extracted antigen | 24000 | 4250 | 5.6 |
| Partially purified antigen | 24000 | 2370 | 10.1 |

**Table 1**

| (2) Purification from HCA-200L | | | |
|---|---|---|---|
| Fractions | Total Antigen Activity (titer) | Total amount of Protein (µg) | Specified Activity of Antigen (titer/µg) |
| passed-through | 0 | 760 | -- |
| 0 - 8% | 0 | 110 | -- |
| 8 - 16% | 2300 | 120 | 12.1 |
| 16 - 24% | 9220 | 140 | 65.0 |
| 24 - 32% | 4610 | 180 | 25.0 |
| 32 - 40% | 580 | 360 | 1.6 |
| 40% or more | 0 | 700 | -- |
| Total of 8-40% | 16130 | 440 | 36.7 |
| Extracted antigen | 24000 | 4250 | 5.6 |
| Partially purified antigen | 24000 | 2370 | 10.1 |

### Example 2 TPHA

The purified antigen obtained in Example 1 was carried on sheep erythrocyte for confirming the effect of the reagent obtained by the process according to the invention by TPHA.

### 1. Materials

The same materials as used in Example 1 were used if unspecified. The buffer was prepared by the same manner as in Example 1.
(1) Buffer
(a) 0.15M sodium phosphate buffer (pH:7.4):
The buffer was prepared by mixing 0.15M sodium dihydrogen phosphate (2 hydrate) with 0.15M disodium hydrogen phosphate (12 hydrate) so as to show pH 7.4.
(b) Physiological saline solution:
The saline solution was prepared by dissolving sodium chloride (9.0 g) in purified water (1000 g).
(c) McIlvaine buffer (hereinafter abbreviated to McI):
McI was prepared by mixing 0.10M citric acid with 0.20M disodium hydrogen phosphate (12 hydrate) so as to show pH 6.5.
(d) 1% OG/McI:
The above-identified solution was obtained by dissolving OG (1% W/V) in McI.
(e) PHA buffer:
The above-identified buffer was prepared by mixing the solutions and reagents described as follows (The amount is expressed per 1000 ml of buffer.)

| | |
|---|---|
| Rabbit normal serum | 30ml |
| Sheep erythrocyte stroma | 10ml |
| Sodium azide | 1g |
| 0.15M sodium phophate buffer (pH 7.4) | 100ml |
| Physiological saline | 860ml |

(2) Reagents
(a) Tannic acid was bought from Nacalai Tesque, Inc.
(b) Fixed sheep erythrocyte was the one immobilized with glutaraldehyde.
(c) TPHA kits commercially available Serodia TP (Fuji Rebio Inc.) and Seroclit TP (The Chemo- Sero-Therapeutic Research Institute) were used.

(3) Serum Samples
(a) Syphilis-positive control
   Three control sera (G₁, G₂ and G₃) were used, those of which were collected from fully cured advanced syphilitic patients. These control sera were supposed to contain a lot of IgG antibodies. Further, three control sera (M₁, M₂ and M₃) were used, those of which were collected from primary syphilitic patients, i.e., three to five weeks after the infection. The latter controls were supposed to contain few Ig-G antibodies, but Ig-M antibodies.
(b) Normal control (Syphilis-negative control):
   Three control sera (N₁, N₂ and N₃) were used, those of which were observed to show nonspecific reaction with the TPHA kit commercially available and further found not to be syphilic by FTA-ABS.
(c) Anti-rabbit tissue antiserum:
   A normal rabbit testis sliced and solubilized with 1% OG was immunized to a goat for obtaining the above-identified serum.
(d) Anti-Reiter strain antiserum:
   Nonphthogenic treponemes, Treponema phagedenis (biotype Reiter), were solubilized with 1% OG. This solution was immunized to a goat for obtaining the above-identified serum.

(4) TP antigen
The extracted antigen, partially purified antigen and HAp purified antigen as obtained in Example 1 were used. Table 2 shows the antigen activity and protein concentration of each antigen solution.

**Table 2**

| Antigen | Antigen Activity (titer/ml) | Protein cocn. (µg/ml) | Specified Activity (titer/µg) |
|---|---|---|---|
| Extracted Antigen | 2048 | 365 | 5.6 |
| Partially purified antigen | 1024 | 101 | 10.1 |
| (HAp Purification) Bio-Gel® HTP | 2048 | 77.0 | 26.6 |
| HCA-200L | 1536 | 41.9 | 36.7 |

### 2. Method

(1) Blood cell processing method
(a) The fixed sheep blood cells were washed four times with a physiological saline by using centrifugation for 5 minutes at 700 x g, which was suspended in a physiological saline to have solid content of 6%. A solution (tannic acid in physiological saline, 120µg/ml) was added to the blood cell suspension, followed by stirring.
(b) After stirring, the resultant solution was washed twice with a physiological saline and once with McI, and then suspended in McI to have solid content of 6%. Immediately, the resultant solution was used for antigen sensitization.
(c) TP antigen solution (the extracted antigen solution, partially purified antigen solution or HAp purified solution) was in advance dialyzed against 1% OG/McI. The obtained solution was adjusted as described in Table 3 to serve as the sensitizing solution. The sensitizing solution A was adjusted to have antigen activity of 100 (titer/ml). The sensitizing solution B was adjusted to have protein concentration of 10µg/ml. One volume of the sensitizing solution was added to one volume of the blood cell suspension as described at (b) and the mixture was stirred for 1 hour at 25°C.

**Table 3**

| | Sensitizing Solution A (Sensitization with a predetermined antigen amount) | | Sensitizing Solution B (Sensitization with a predetermined protein amount) | |
|---|---|---|---|---|
| Antigen | Antigen Solution (ml) | 1%OG/McI (ml) | Antigen Solution (ml) | 1%OG/McI (ml) |
| Extracted | 0.049 | 0.951 | 0.027 | 0.973 |
| Partially purified | 0.098 | 0.902 | 0.099 | 0.901 |
| (HAp purification) Bio-Gel® HTP | 0.049 | 0.951 | 0.130 | 0.870 |
| HCA-200L | 0.065 | 0.935 | 0.239 | 0.761 |

(d) The sensitized blood cells were washed twice with a physiological saline and suspended in PHA buffer to have blood cell solid content of 0.2%. After standing for 3 hours at room temperature, the resultant suspension was used for assay.
(2) TPHA assay method
(a) 100µl of PHA buffer was dispensed in each well of the microtiter plate, and 25µl in other wells.
(b) 25µl of each control was dispensed in each well of the microtiter plate, which was serially diluted 2¹ to 2ⁿ on the plate.
(c) The blood cell suspension prepared in (1) was shaken to obtain a homogeneous suspension. 75µl of the suspension was dispensed in each well.

(4) The plate was vibrated to mix sufficiently. Thereafter, the plate was covered with an empty plate in order to prevent evaporation and then incubated at room temperature. The determination was conducted after 2 hours.
(5) The plate was placed on white paper, observing hemagglutination by visual observation. The maximum dilution (20, 40, 80, ....) exhibiting agglutination was defined as the antibody titer.

### 3. Result

Table 4 shows the results of determination of hemagglutination on each control and of measurement of antibody titer by using the sensitized blood cell obtained by the above method.

As shown in Table 4, three primary syphilitic sera which were all negative with the sensitized blood cell using the extracted antigen were positive with the sensitized blood cell using the HAp purified antigen of Example 1. Normal control sera exhibiting non-specific reaction by the TPHA kit showed no agglutination. Further, the sensitized blood cell using the present invention exhibited no agglutination with the anti-rabbit tissue antiserum and anti-Reiter strain-antiserum.

### 4. Conclusion

As is apparent from the result, even a primary syphilis is detectable by using the diagnostic reagent according to the present invention, without giving a false positive result due to the nonspecific reaction.

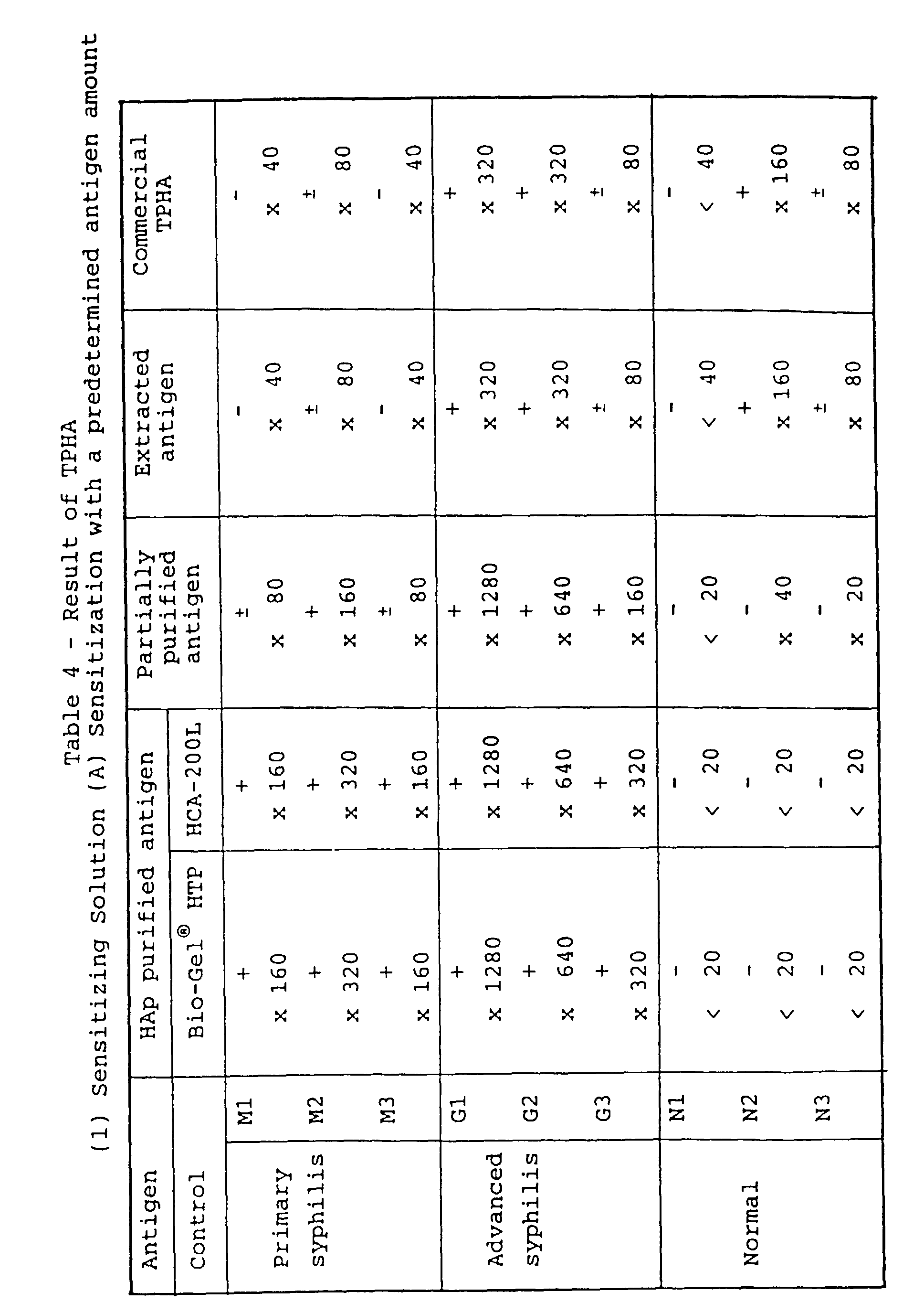

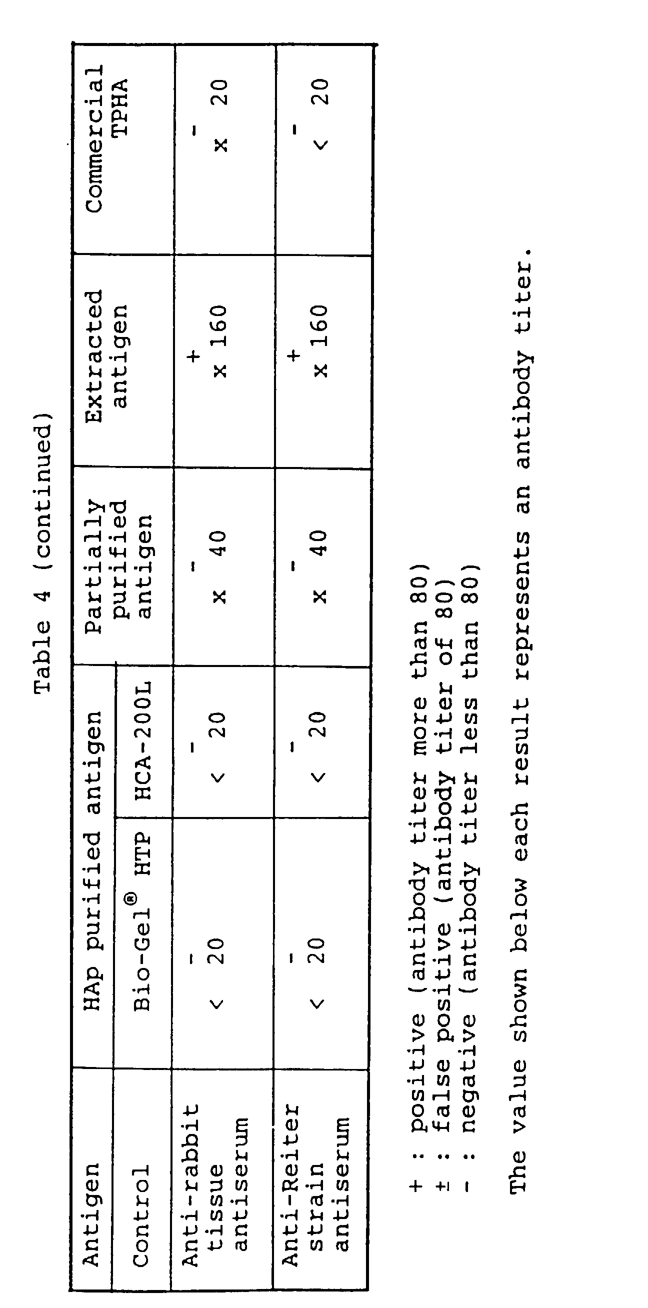

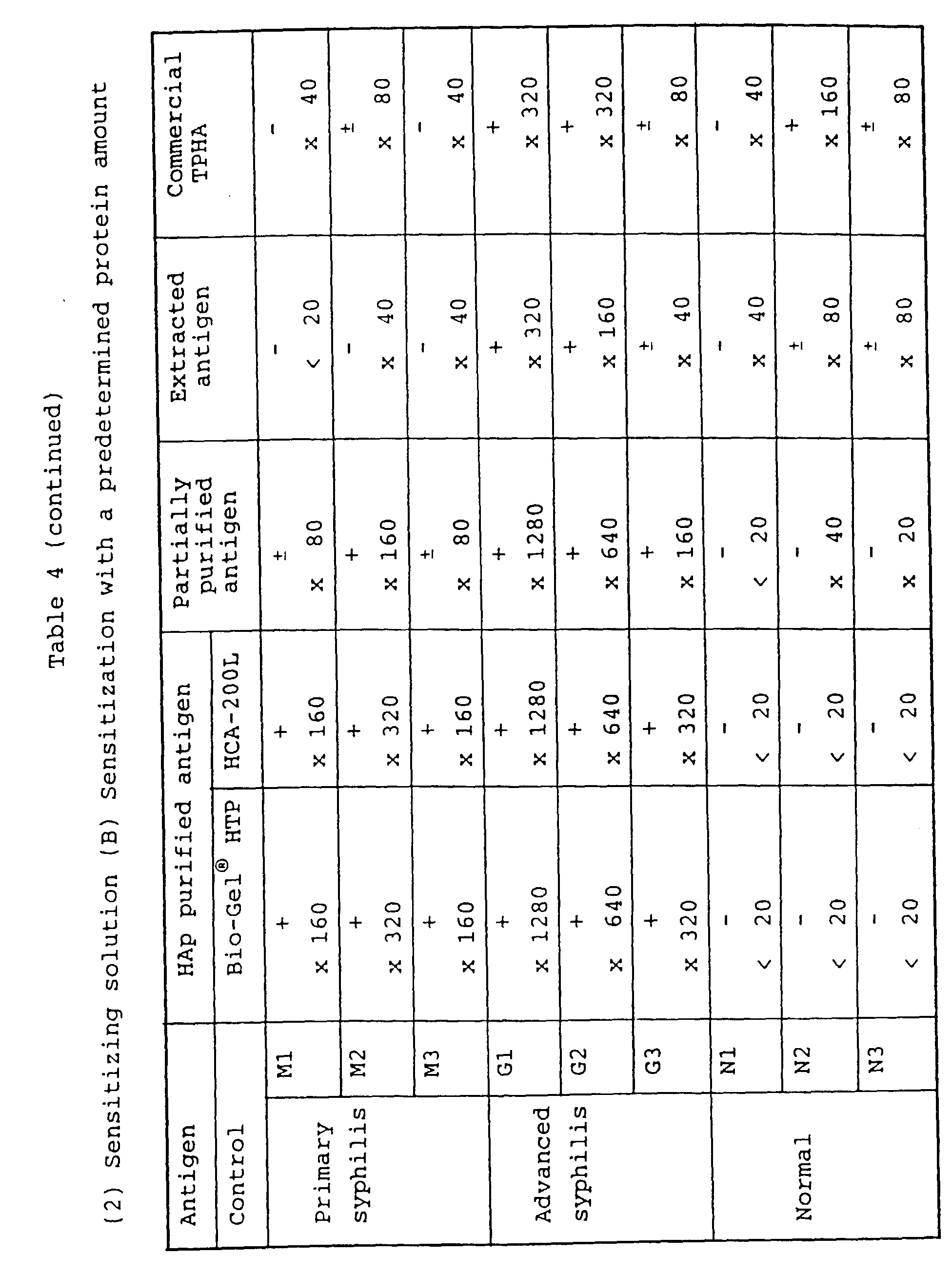

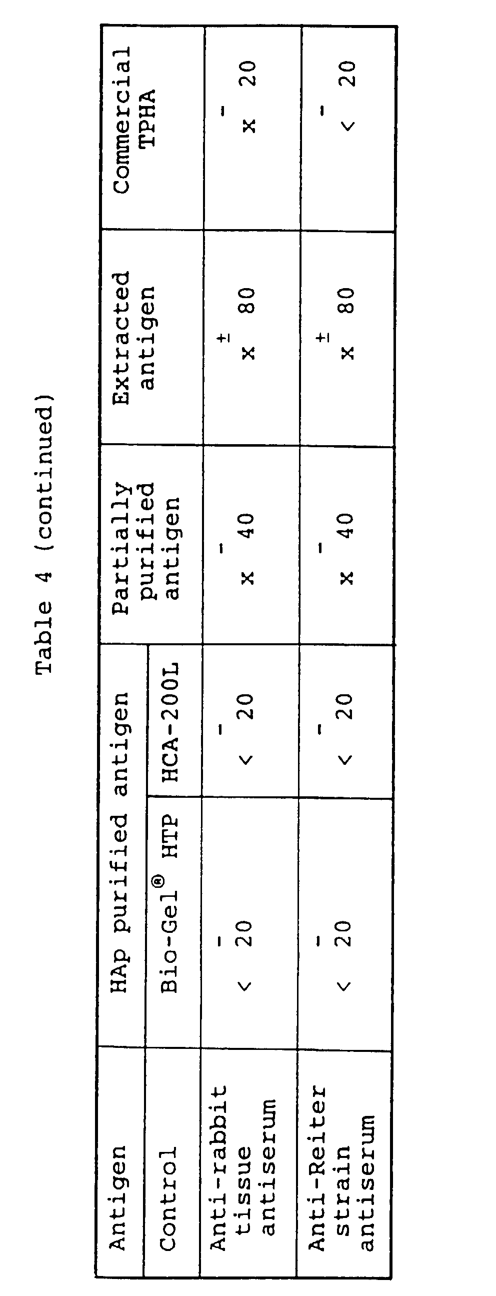

### Example 3 Latex reagent (for measuring method using fully-automatic analyzer)

In the case of detecting anti-treponemal antibodies, the effect of the diagnostic latex reagent as prepared by the present invention was confirmed by measuring the agglutination with the use of a fully-automatic analyzer. Controls as those used in Example 1 and Example 2 were prepared by the same manner as in Example 1 and Example 2 if unspecified.

### 1. Preparation of reagent and control

### (1) Latex:

Polystyrene latex (solid:10%) of 0.400µm (Sekisui Chemical Co., Ltd.) was used.

### (2) PBS (pH:7.4):

A solution of 0.02M phosphate and 0.15M sodium phosphate (2 hydrate), disodium phosphate (2 hydrate) and sodium chloride, to which NaN₃ (as preservative) was added at 0.1%.

### (3) NaCl-PBS (pH: 6.5):

A buffer was prepared from sodium phosphate (2 hydrates), disodium phosphate (2 hydrates) and sodium chloride to make phosphate concentration of 0.02M, NaCl concentration of 1.00M and pH 6.5.

### (4) 100mM NaPB:

A solution of 100mM NaPB (pH:7.5) were prepared from disodium hydrogen phosphate (anhydrous) and sodium dihydrogen phosphate (12 hydrate), to which NaN₃ was added at 0.1%.

### (5) 1% BSA-NaPB:

The above-identified solution was prepared to have 1% BSA in 100mM NaPB.

### (6) 5% BSA-NaPB:

The above-identified solution was prepared to have 5% BSA in 100mM NaPB.

### (7) Diluent:

The diluent was prepared by dissolving polyethylene glycol (average molecular weight:500,000, Wako Pure Chemical Industries, Ltd.) in 0.25% (W/V) in 5% BSA-NaPB.

### (8) Instrument:

Measurement was performed on Hitachi 7050 Type, a fully-automatic analyzer.

### 2. Method

### (1) Preparation of antigen sensitizing solution

To each antigen solution having antigen titer and protein concentration shown in Table 2 was added a mixture of 10mM KPB, of NaCl-PBS and 1% OG in the amount shown in Table 5. The obtained solution was defined as the sensitizing solution (the antigen solution for immobilization) having pH 5.4 to 6.5. The sensitizing solution A was prepared to have antigen activity of 250 (titer/ml), while the sensitizing solution B was prepared to have protein concentration of about 25µg/ml.

**Table 5**

| Sensitizing solution A (Sensitization with a predetermined antigen amount) | | | |
|---|---|---|---|
| Antigen | Antigen Solution (ml) | 1% OG/KPB pH 6.0 (ml) | NaCl/PB (ml) |
| Extracted antigen | 0.049 | 0.251 | 0.100 |
| Partially purified antigen | 0.098 | 0.202 | 0.100 |
| (HAp purification) HCA-200L | 0.049 | 0.251 | 0.100 |
| Bio-Gel® HTP | 0.065 | 0.235 | 0.100 |

| Sensitizing solution B (Sensitization with a predetermined protein amount) | | | |
|---|---|---|---|
| Antigen | Antigen Solution (ml) | 1% OG/KPB pH 6.0 (ml) | NaCl/PB (ml) |
| Extracted antigen | 0.027 | 0.273 | 0.100 |
| Partially purified antigen | 0.099 | 0.201 | 0.100 |
| (HAp purification) HCA-200L | 0.130 | 0.170 | 0.100 |
| Bio-Gel® HTP | 0.239 | 0.061 | 0.100 |

### (2) Immobilization of treponemal antigen

Polystyrene Latex (100µl) (solid content of 10% by weight) was stirred by a magnetic stirrer in an incubator at 4°C, with which the treponemal antigen solution (400µl) prepared in (1) was quickly mixed and stirred for 1 hour at 4°C. After the addition of 1% BSA (5 ml), the resultant mixture was stirred for 1.5 hours at 4°C and then centrifuged for 1 hour at 15,000 rpm. To the obtained pellets was added again 1% BSA-NaPB (5 ml). The resultant mixture was centrifuged by the same manner as described above and washed. 1% BSA-NaPB (5 ml) was added to the final pellets and sufficiently dispersed, thereby affording latex reagent having solid content of 0.2%. Thus obtained Latex reagent was kept at 4°C.

### (3) Parameters of Hitachi 7050 Type, a fully-automatic analyzer

| | |
|---|---|
| Sample content | 20µl (serum) |
| R1 content | 50µl (Latex reagent) |
| R2 content | 350µl (diluent) |
| Wavelength | 570nm |

### (4) Measuring method

The difference of the absorbance between 80 seconds after the beginning of the measurement and 320 seconds after the beginning of the measurement was taken. 10⁴ of this difference was defined as the variation at O.D. 570.

### 3. Result

The reaction of each control with the Latex reagent prepared from the TP antigen solution by the manner described above was measured as the variation of turbidity at O.D. 570. Table 6 shows the results.

As shown in Table 6, three primary syphilitic sera which were all negative with the latex reagents using the extracted antigen and partially purified antigen exhibited sensitivity sufficient for determining to be positive with the latex reagent using the purified TP antigen of Example 1. Three advanced syphilitic sera exhibiting nonspecific reaction with the TPHA kit showed no agglutination with the latex reagent using the purified TP antigen of Example 1.

Further, the latex reagent of the present invention did not exhibit a turbidity for determining to be positive with the anti-rabbit tissue antiserum and anti-Reiter strain antiserum.

### 4. Conclusion

As is apparent from the result, the reagent prepared from the purified antigen according to Example 1 is more reactive, i.e, high-sensitive compared to there from the conventional extracted antigen. As a result, even primary syphilis, which cannot be detected by the conventional extracted antigen can be detected by using the HAp purified antigen.

Further, the use of the purified antigen can detect primary syphilis, which cannot be detected by the commercially available TPHA kit, and also does not give the false positive result by the nonspecific reaction.

**Table 6**

| Result with a latex reagent | | | | | |
|---|---|---|---|---|---|
| Sensitizing Solution (A) Sensitization with a predetermined antigen amount | | | | | |
| Antigen | | HAp purified antigen | | Partially purified antigen | Extracted antigen |
| Control | | Bio-Gel® HTP | HCA-200L | | |
| Primary syphilis | M1 | 323 | 639 | 157 | 44 |
| | M2 | 857 | 1433 | 184 | 103 |
| | M3 | 646 | 626 | 108 | 38 |
| Advanced syphilis | G1 | 2101 | 3289 | 1624 | 678 |
| | G2 | 1655 | 2273 | 683 | 275 |
| | G3 | 849 | 1274 | 154 | 110 |
| Normal | N1 | 4 | 2 | 19 | 55 |
| | N2 | 3 | 5 | 16 | 21 |
| | N3 | 2 | 7 | 19 | 21 |
| Anti-rabbit tissue antiserum | | 3 | 3 | 40 | 105 |
| Anti-Reiter strain antiserum | | 5 | 9 | 32 | 97 |

| Sensitizing Solution (B) Sensitization with a predetermined protein amount | | | | | |
|---|---|---|---|---|---|
| Antigen | | HAp purified antigen | | Partially purified antigen | Extracted antigen |
| Control | | Bio-Gel® HTP | HCA-200L | | |
| Primary syphilis | M1 | 1231 | 1710 | 161 | 16 |
| | M2 | 3144 | 3882 | 171 | 41 |
| | M3 | 1231 | 1695 | 122 | 21 |
| Advanced syphilis | G1 | 7812 | 7540 | 1585 | 291 |
| | G2 | 6129 | 6148 | 652 | 100 |
| | G3 | 3102 | 3225 | 171 | 65 |
| Normal | N1 | 5 | 3 | 317 | 5 |
| | N2 | 2 | 2 | 15 | 3 |
| | N3 | 3 | 7 | 19 | 10 |
| Anti-rabbit tissue antiserum | | 9 | 6 | 39 | 66 |
| Anti-Reiter strain antiserum | | 5 | 8 | 41 | 35 |

### Example 4 Assay for syphilis antibody by ELISA method

The effect of the reagents of the present invention was confirmed by ELISA method.

### 1. Preparation of reagent and control

The following reagents and controls were prepared for use. The same reagents and controls as those used in Examples 1, 2 and 3 were prepared by the same manner as in Examples 1, 2 and 3.

### (1) TP antigen solution

Used antigen solution were the extracted antigen, partially purified antigen and HAp purified antigen obtained in Example 1.

### (2) Control

The controls used in Example 2 were diluted by a hundred fold with 1% BSA/PBS.

### (3) Peroxidase labelled anti-goat Ig-G

Peroxidase labelled anti-goat Ig-G (originated from sheep) (Miles Laboratories Co.) was diluted by a thousand fold with 1% BSA/PBS without NaN₃.

### (4) Peroxidase labelled anti-human Ig-G and Ig-M

Peroxidase labelled anti-goat Ig-G and Ig-M (originated from sheep) (Miles Laboratories Co.) were diluted by a thousand fold with 1% BSA/PBS without NaN₃.

### (5) Microtiter plate:

A microtiter plate having 96 wells (Nunc Co., flat bottom for ELISA) was used.

### (6) Peroxidase substrate:

o-Phenylenediamine (dihydrochloride) (2 mg/ml) and aqueous hydrogen peroxide (0.03%) were dissolved in a phosphoric acid-citric acid buffer (pH 5.0). The substrate was prepared immediately before being used.

### (7) Stop solution:

1N sulfuric acid solution was used as the stop solution of enzyme reaction.

### 2. Method

### (1) Preparation of antigen solution:

By the same manner as in Example 3, to each antigen solution was added a mixture of 10mM KPB, NaCl-PB and 1% OG in the amount shown in Table 7.

### (2) Immobilization of the TP antigen

The TP antigen solution prepared in (1) was dispersed into the microtiter plate in an amount of 50µl and incubated for 1 hour at room temperature.

After the incubation, the excess TP antigen solution was removed and washed three times with 1% BSA/PBS (200µl) under suction. Thereafter, 1% BSA/PBS (200µl) was added to the resultant and incubated for 1 hour at room temperature for effecting blocking. The plate to which blocking was completed was used for antigen-antibody reaction.

### (3) Antigen-antibody reaction

As a first antibody, the control diluted hundredfold with 1% BSA/PBS was pipetted into each well in an amount of 100µl. As a control, the control was similarly pipetted into each well to which 1% BSA/PBS was added instead of the antigen. After incubated for 1 hour at room temperature, the solution was removed under suction and washed three times with 1% BSA/PBS (200µl) under suction.

Then, the peroxidase labelled anti-human Ig-G and anti-human Ig-M were pipetted in an amount of 100µl into each well into which the primary syphilis sera, advanced syphilis sera and syphilis-negative sera were pipetted. Further, the anti-goat Ig-G was pipetted in an amount of 100µl into each well into which the anti-Reiter strain antiserum and anti-rabbit tissue antiserum were pipetted. After the incubation for 1 hour at room temperature, each well was sucked and washed three times with 200µl of 1% BSA/PBS. Immediately after washing, enzyme activity bound to each well was measured.

### (4) Enzymatic reaction

100µl of peroxidase substrate was added to each well and the plate was incubated for 15 minutes at room temperature. As a substrate blank, the substrate was pipetted into each well not containing antigen, first antibody or second antibody. After the incubation, 1N stop solution (100µl) was added to stop the enzymatic reaction. After stopping the reaction, the absorbance at 492nm was measured with a microtiter plate reader (MTP-100, Corona Electric Co., Ltd.), in comparison with the substrate blank.

### 3. Result

Table 8 shows the results of the absorbance at 492nm.

Three primary syphilitic sera increase in detection sensitivity of IgM by using the purified antigen of Example 1. Three syphilis-negative controls exhibit falsely positive (the value of more than O.D. 0.057 is determined to be positive) by using the extracted antigen, while do not exhibit positive by using the purified antigen of Example 1.

Moreover, the purified antigen of Example 1 did not show a value for determining to be positive with the anti-rabbit tissue antiserum and anti-Reiter strain antiserum.

### 4. Conclusion

As is apparent from the above result, the purified antigen used in the process according to the present invention can detect even primary syphilis, which can not be detected by ELISA method using the conventional extracted antigen, and also does not give a false positive result by the nonspecific reaction.

### Example 5 Latex reagent

The partially purified antigen solution obtained in Example 1 was used which was dissolved in 10mM potassium phosphate buffer (pH:6.0) containing 1% OG.

Sensitizing solution having protein concentration of 30µg/ml, predetermined OG concentration and predetermined pH was prepared by diluting the antigen solution with buffer or adding OG to the antigen solution.

Latex reagent was prepared by the same manner as in Example 3.

Used control was obtained by diluting rabbit serum with phosphate buffered saline [0.02M phosphate buffer, 0.13M sodium chloride (pH:7.4) and 0.1% sodium azide] containing 1% BSA.

The controls were measured with the obtained latex reagent by the same manner as in Example 3. Table 7 shows the result.

**Table 7**

| Sensitizing solution | | % for dilution % | Variation | |
|---|---|---|---|---|
| cocn. of OG (%) | pH | | syphilis-negative | syphilis-positive |
| 0.5 | 5.0 | 400 | 8.0 | 205.2 |
| | | 200 | 6.0 | 423.0 |
| | | 100 | 7.0 | 1024.0 |
| 0.5 | 6.0 | 400 | 3.0 | 237.0 |
| | | 200 | 5.0 | 481.2 |
| | | 100 | 13.0 | 1312.0 |
| 0.5 | 7.0 | 400 | 10.0 | 225.0 |
| | | 200 | 9.0 | 453.6 |
| | | 100 | 7.0 | 1200.0 |
| 0.5 | 7.5 | 400 | 4.0 | 186.6 |
| | | 200 | 7.0 | 372.0 |
| | | 100 | 12.0 | 960.0 |
| 0.025 | 6.0 | 400 | 6.0 | 198.0 |
| | | 200 | 11.0 | 420.0 |
| | | 100 | 10.0 | 1200.0 |
| 1.0 | 6.0 | 400 | 7.0 | 177.0 |
| | | 200 | 8.0 | 372.0 |
| | | 100 | 6.0 | 1088.0 |
| 2.0 | 6.0 | 400 | 3.0 | 192.0 |
| | | 200 | 6.0 | 378.0 |
| | | 100 | 9.0 | 1016.0 |
| note: Controls are philis-negative and syphilis-positive (10,000 titer) rabbit serum. Four hundred fold, two hundred fold and one hundred fold the syphilis-positive serum respectively correspond to 25, 50 and 100 titer. | | | | |

## Claims

1. A process for preparing a diagnostic reagent for syphilis which comprises:
a) contacting an antigen originated from Treponema pallidum with an inert carrier having at least partial hydrophobicity on its surface in an aqueous medium having a pH from 4.5 to 7.7 and containing 0.01 to 2.5 % by weight of a surfactant;
b) washing the carrier to remove any remaining surfactant,
wherein said surfactant is octylglucopyranoside.

2. The process of claim 1 wherein the aqueous medium contains 0.02 to 2.10 weight % of octylglucopyranoside.

3. The process of any of claims 1 or 2 wherein the aqueous medium has a pH of from 4.9 to 7.1.

4. The process of claim 3 wherein the aqueous medium has a pH of from 5.4 to 6.5.

5. A process of any of claims 1 to 4 wherein the aqueous medium is phosphate buffer.

6. A process of any of claims 1 to 5 wherein the washing of step b) is conducted with a buffer containing bovine serum albumine or saline solution.

7. A process of any of claims 1 to 6 wherein the inert carrier is latex particles or erythrocytes treated with tannic acid.

8. A process of any of claims 1 to 6 in which the antigen is a purified antigen obtained by a process which comprises adsorbing an extract originated from Treponema pallidum on a hydroxyapatite gel, followed by elution, while an aqueous medium is used.

## Patentansprüche

1. Verfahren zur Erstellung eines diagnostischen Reagenses für Syphilis, umfassend:
a) Kontaktierung eines Antigens, das von Treponema pallidum herrührt, mit einem inerten Träger, der auf seiner Oberfläche eine mindestens teilweise Hydrophobizität aufweist, in einem wäßrigen Medium mit einem pH-Wert von 4,5 bis 7,7, das 0,01 bis 2,5 Gew.-% eines oberflächenaktiven Mittels enthält;
b) Waschen des Trägers zur Entfernung von zurückgebliebenem oberflächenaktivem Mittel, wobei das genannte oberflächenaktive Mittel Octylglucopyranosid ist.

2. Verfahren nach Anspruch 1, dadurch **gekennzeichnet**, daß das wäßrige Medium 0,02 bis 2,10 Gew.-% Octylglucopyranosid enthält.

3. Verfahren nach Anspruch 1 oder 2, dadurch **gekennzeichnet,** daß das wäßrige Medium einen pH-Wert von 4,9 bis 7,1 hat.

4. Verfahren nach Anspruch 3, dadurch **gekennzeichnet,** daß das wäßrige Medium einen pH-Wert von 5,4 bis 6,5 hat.

5. Verfahren nach einem der Ansprüche 1 bis 4, dadurch **gekennzeichnet**, daß das wäßrige Medium ein Phosphatpuffer ist.

6. Verfahren nach einem der Ansprüche 1 bis 5, dadurch **gekennzeichnet,** daß das Waschen der Stufe b) mit einem Puffer durchgeführt wird, der ein Rinderserumalbumin oder eine Kochsalzlösung enthält.

7. Verfahren nach einem der Ansprüche 1 bis 6, dadurch, **gekennzeichnet,** daß der inerte Träger aus Latexteilchen oder Erythrozyten, die mit Gerbsäure behandelt worden sind, besteht.

8. Verfahren nach einem der Ansprüche 1 bis 6, dadurch **gekennzeichnet**, daß das Antigen ein gereinigtes Antigen ist, erhalten durch ein Verfahren, das die Adsorption eines Extrakts, der von Traponema pallidum herrührt, auf einem Hydroxyapatitgel und die anschließende Elution unter Verwendung eines wäßrigen Mediums umfaßt.

## Revendications

1. Procédé de préparation d'un réactif de diagnostic de syphilis qui comprend :
(a) la mise en contact d'un antigène provenant de Treponema pallidum avec un support inerte ayant au moins une hydrophobicité partielle sur sa surface, dans un milieu aqueux à un pH compris entre 4,5 et 7,7 et contenant 0,01 à 2,5 % en poids d'un surfactant ;
(b) le lavage du support pour éliminer tout le surfactant résiduel,
et dans lequel ledit surfactant est l'octylglucopyranoside.

2. Procédé selon la revendication 1, dans lequel le milieu aqueux contient 0,02 à 2,10 % en poids d'octylglucopyranoside.

3. Procédé selon l'une quelconque des revendications 1 ou 2, dans lequel le milieu aqueux a un pH compris entre 4,9 et 7,1.

4. Procédé selon la revendication 3, dans lequel le milieu aqueux a un pH compris entre 5,4 et 6,5.

5. Procédé selon l'une quelconque des revendications 1 à 4, dans lequel le milieu aqueux est un tampon phosphate.

6. Procédé selon l'une quelconque des revendications 1 à 5, dans lequel le lavage de l'étape (b) est effectué avec un tampon contenant de l'albumine de sérum bovin ou du sérum physiologique.

7. Procédé selon l'une quelconque des revendications 1 à 6, dans lequel le support inerte est constitué de particules de latex ou d'érythrocytes traités par l'acide tannique.

8. Procédé selon l'une quelconque des revendications 1 à 6, dans lequel l'antigène est un antigène purifié obtenu par un procédé qui comprend l'absorption d'un extrait provenant de Treponema pallidum sur un gel d'hydroxyapatite, suivie d'une élution avec utilisation d'un milieu aqueux.
